# EUROPÄISCHE PATENTSCHRIFT

(11) **EP 4 233 785 B1**
(45) Veröffentlichungstag und Bekanntmachung des Hinweises auf die Patenterteilung: **18.02.2026**
(21) Anmeldenummer: 23177662.6
(22) Anmeldetag: 09.11.2012
(51) Int. Cl.: A61C 13/00, A61C 13/01, A61C 13/20

(54) **FRÄSBLOCKSYSTEM UND VERFAHREN ZUR HERSTELLUNG VON TEIL- ODER TOTALPROTHESEN**
MILLING BLOCK SYSTEM AND METHOD FOR THE PREPARATION OF PARTIAL OR TOTAL PROSTHESES
SYSTÈME DE BLOCS DE FRAISAGE ET PROCÉDÉ DE FABRICATION DE PROTHÈSES PARTIELLES OU TOTALES

(30) Priorität: 11.11.2011 DE 102011118320
(43) Veröffentlichungstag der Anmeldung: 30.08.2023
(62) Teilanmeldung aus: 18198191.1
(73) Patentinhaber: Merz Dental GmbH, 24321 Lütjenburg (DE)
(72) Erfinder: Klingenburg, Friedhelm, 24321 Lütjenburg (DE); Frölich, Carola, 89073 Ulm (DE)
(74) Vertreter: Kunz, Herbert

(56) Entgegenhaltungen:
- WO-A1-2010/057584
- DE-A1- 4 025 728

## Beschreibung

Die Erfindung betrifft ein Fräsblocksystem bestehend aus einem ersten Fräsblock für das Obergebiss und einem zweiten Fräsblock für das Untergebiss und ein Verfahren zur Bereitstellung einer Teil- oder Totalprothese.

Aus der EP 0 501 983 B1 bzw. DE 40 25 728 A1 ist bereits ein Verfahren zur Herstellung einer Totalprothese mit einer Ober- und/oder Unterkieferbasis mit Hilfe einer Fräseinrichtung bekannt, wobei die jeweilige Basis aus einem Kunststoffblock ausgefräst wird und die Kontur oder Topographie kennzeichnenden Daten der Mundhöhle verwendet werden. Dies darin beschriebene Verfahren verwendet ferner nach Erstellen der angepassten Basis an die Mundhöhle den weiteren Verfahrensschritt, notwendige Zähne in Wachs auf die Ober- und/oder Unterkieferbasis aufzustellen, wobei die Fertigstellung der Prothese durch Verbinden der Zähne mit der Basis mit Hilfe eines Kaltpolymerisates durchgeführt wird. Daran anschließend erfolgt das sogenannte Reokkludieren, damit dem jeweiligen Patienten entsprechend seiner Kaubewegungen ein optimaler Biss vermittelt wird.

Dieses bekannte Verfahren beinhaltet somit den Nachteil, dass nach Fertigstellung der Ober-und/oder Unterkieferbasis ein individuelles Anpassen an den Patienten durch entsprechendes Aufstellen der Zähne in Wachs erforderlich wird, was wiederum den gesamten zahntechnischen und zahnärztlichen Behandlungsablauf zeit- und kostenintensiv macht.

Aufgabe der folgenden Erfindung ist es somit, ein Fräsblocksystem und ein Verfahren zur Herstellung von Teil- oder Totalprothesen bereitzustellen, welche die Nachteile des Standes der Technik vermeiden. Ferner ist es Aufgabe der folgenden Erfindung, die Arbeitsschritte zur individuellen Anpassung an den Patienten zu verringern.

Erfindungsgemäß wird zur Lösung der Aufgaben ein Fräsblocksystem zur Herstellung von Teil- oder Totalprothesen gemäß Anspruch 1 sowie ein Verfahren gemäß Anspruch 8 zur Herstellung von Teil- oder Totalprothesen aus einem solchen Fräsblocksystem vorgeschlagen. In diesem Sinne ist das Fräsblocksystem hinsichtlich Obergebiss und Untergebiss unabhängig von der Befestigung im Mund ausgestattet.

Mit dieser anmeldungsgemäßen Maßnahme wird erreicht, dass lediglich die Prothesenbasis gefräst bzw. geformt werden muss, und bei korrekter Fräsung entsprechend vorliegender CAD/CAM-Daten der Mundhöhle des Patienten aufgrund bereits auf der Prothesenbasis verankerter künstlich geformter Zähne keine weitere Anpassung erfolgen muss.

Durch entsprechend optimierte Einpassung und Justierung der Prothesenbasis in die Mundhöhle wird somit nur ein individueller Anpassungsschritt erforderlich sein, um den Patienten optimal zu versorgen.

Aufgrund des anmeldungsgemäßen Vorhandenseins eines Fräsblocksystems, bei dem eine entsprechende Kieferform zur bearbeitenden Prothesenbasis mit künstlich geformten Zähnen vorliegt, muss weder eine separate Bissnahme für eine Kieferrelationsbestimmung, noch eine zeit- und kostenintensive Anprobe vorgenommen werden.

Mit der anmeldungsgemäßen Maßnahme ist somit ein nachträgliches Einsetzen in einem Artikulator zur Bestimmung der Positionierung der Zähne nicht erforderlich, da die künstlich geformten Zähne bereits entsprechend gegebener Grunddaten für die Okklusionsstellung vorbereitet sind.

Mit dem anmeldungsgemäßen Verfahren zur Bereitstellung einer Teil- oder Totalprothese ist es somit möglich, dass aufgrund des Vorhandenseins von in Okklusionsstellung vorliegenden Zahnober- und unterreihen in Gebissform mindestens drei unterschiedliche Fräsblocksysteme mit unterschiedlichen Größenverhältnissen in der Gebissform und Größe der Zähne bereitgestellt werden können, um nahezu alle Patienten versorgen zu können. Mit dieser Maßnahme wird ein zeit- und kosteneffizientes Herstellungsverfahren erzielt und dem Zahnarzt und/oder dem Zahntechniker in allen Ländern eine vollständige Versorgung der Bevölkerung ermöglicht.

Im Unterschied zu den bekannten Dentalprothesen bzw. deren Herstellungsverfahren richtet sich das dererfindungsgemäße Fräsblocksystem auf eine einstückige Anordnung mit einem ersten Bereich, welcher eine entsprechend der Kieferform bearbeitbare Prothesenbasis aufweist und einem zweiten Bereich mit einer vorbestimmten, bzw. standardisierten Anordnung künstlich geformter Zähne oder Zahnreihen für ein Ober- bzw. Unterkiefergebiss. Damit wird als Vorstufe zur Dentalprothese ein Fräsblock bereitgestellt, der besonders kosten- und zeitsparend lediglich in seinem ersten Bereich, nämlich der Prothesenbasis, zur Anpassung an die Kieferform bearbeitbar, d.h. fräsbar ist. Das erfindungsgemäße Fräsblocksystem umfasst zumindest einen ersten Fräsblock für das Obergebiss und einen zweiten Fräsblock für das Untergebiss, d.h. die Vorstufen einer Teil- oder Totalprothese, welche im jeweils zweiten Bereich des ersten und/oder zweiten Fräsblocks eine vorbestimmte bzw. standardisierte und nach gängigen Okklusionsprinzipien aufgestellte Anordnung künstlich geformter Zähne oder Zahnreihen für das Ober- bzw. Unterkiefergebiss aufweist.

Eine aus dem Stand der Technik bekannte Mehrzahl von Vorformlingen mit verschiedenen Farben, Größen- und Formenkombinationen für den Schleimhautbereich und Zahnbereich zur Herstellung von Zahnersatz wird mit vorliegendem Erfindungsgegenstand durch die vorbestimmte bzw. standardisierte Anordnung künstlich geformter, einpolymerisierter Zähne oder Zahnreihen für das Ober- bzw. Unterkiefergebiss erheblich vereinfacht. Vorteilhaft ist anmeldungsgemäß, dass lediglich nur die jeweilige, dem Schleimhautbereich zugeordnete Prothesenbasis zu fräsen ist.

Darüber hinaus handelt es sich bei der vorbestimmten bzw. standardisierten und nach gängigen Okklusionsprinzipien aufgestellten Anordnung künstlich geformter Zähne oder Zahnreihen für das Ober- bzw. Unterkiefergebiss um nicht-individualisierte, vorgefertigte Zahnersatzteile, die keiner weiteren Nachbearbeitung bedürfen.

Die vorbestimmte bzw. standardisierte und nach gängigen Okklusionsprinzipien aufgestellten Anordnung künstlich geformter Zähne oder Zahnreihen für das Ober-bzw. Unterkiefergebiss erfolgt nach einer Auswahl aus vorgefertigten Zahneratzteilen, d.h. künstlich geformter Zähne oder Zahnreihen nach vorgegebenen und bekannten Aufstellsystemen.

Weitere vorteilhafte Weiterbildungen der Erfindung sind Gegenstand der Unteransprüche.

Sind darüber hinaus die künstlich geformten Zähne und die Prothesenbasis einstückig ausgebildet, so kann bei der Herstellung des Fräsblockes nahezu eine Guss- bzw. Pressform verwendet werden, die somit auch jeweils nur ein Material für den Fräsblock verwendet, welches vorteilhaft sowohl ein geringeres Allergiepotential als auch eine höhere Verbundfestigkeit aufweist.

Ist vorteilhafterweise der Fräsblock zweiteilig ausgestaltet, so ist die Möglichkeit gegeben, dass der erste Bereich, der entsprechend der Kieferform bearbeitet wird, standardisiert hergestellt werden kann und mit unterschiedlichen, ebenfalls schon auf einem zweiten Bereich aufgestellten Zähnen, welche unterschiedliche Größen und Gegebenheiten aufweisen, zusammengefügt werden kann. Mit der Weiterbildung, dass die Prothesenbasis flexibel ausgestaltet wird, kann einer vereinfachten Einpassung in die Mundhöhle bzw. Anpassung an die Kieferform des jeweiligen Patienten Rechnung getragen werden.

Als besonders vorteilhafte Materialien für den Fräsblock bzw. für das Fräsblocksystem, d. h. Prothesenbasis und/oder Zähne, bieten sich u. a. sogenannte PMMA-Kunststoffe, ebenso als Thermoplaste, organisch als auch anorganisch gefüllte Kunststoffe und auch allergiearme Kunststoffe an. Zusätzlich ist es ebenfalls vorteilhaft, jedwede Form von Keramiken, insbesondere Glaskeramiken, leuzitverstärkte Keramik, aluminiumverstärkte Keramik, Oxidkeramik, Zirkonoxidkeramik, Infiltrationskeramik, Feldspatkeramiken, Lithium-Disilikat-Keramik, zu verwenden. Denkbar ist es ebenfalls, Materialien zu verwenden, die gesintert werden, die jedoch noch einen weiteren Fertigungsschritt benötigen. Verwendbar ist der Fräsblock bzw. das Fräsblocksystem auch bei kombiniertem Zahnersatz.

Sind die Zähne entsprechend einer Okklusionsstellung bereits ausgerichtet, wird somit nur durch entsprechende Fräsung der Prothesenbasis die vollständige Einpassung in die Mundhöhle des Patienten vorgenommen. Der Patient kann damit sichergehen, dass bei entsprechender Anpassung in die Mundhöhle bzw. an den Ober- bzw. Unterkiefer eine optimale Okklusionsstellung bereitsteht und somit vorteilhaft eine hinsichtlich des Bissverhaltens uneingeschränkt funktionsfähige Totalprothese vorliegt. Aufgrund des anmeldungsgemäßen Fräsblockes bzw. Fräsblocksystems und dem entsprechenden Verfahren zur Herstellung von Teil- oder Totalprothesen ist es beispielsweise möglich, mindestens drei unterschiedliche Größen bereitzustellen, mit denen nahezu für jeden Patienten entsprechend seiner Gesichtsform und Größe der Mundhöhle eine vollständige Totalprothese bereitgestellt werden kann. Aufgrund der Einfachheit der Anpassung der Totalprothese sind somit die Arbeitsschritte zur Anpassung in die Mundhöhle erheblich reduziert und damit auch kostengünstiger. Je nach Erfahrungswerten kann bei bestimmten Bevölkerungsgruppen das Fräsblocksystem auch nur zwei Arten oder mehr beinhalten.

Hinsichtlich der Vorbereitung und Aufstellung der künstlich geformten Zähne sind bei dem anmeldungsgemäßen Fräsblocksystem und dem entsprechenden Verfahren alle hierzu möglichen, bislang bekannten Aufstellsysteme einsetzbar, wie z. B. die sogenannten Uraufstellsysteme gemäß Gysi, Gerber und Schreinemaker. An die Aufstelltechniken von Gysi und Gerber anlehnende Aufstelltechniken gemäß APF und APF NT und TiF sind ebenfalls vorteilhaft einsetzbar. Gemäß Staub Cranial wird eine mathematisch berechnende Aufstelltechnik verwendet, die aufgrund von erstellten Messungen die ursprüngliche Position der Zähne bestimmt und dort entsprechend wieder positioniert.

Das Fräsblocksystem ist insbesondere auch bei der Herstellung von Teilprothesen einsetzbar, insbesondere, wenn zumindest gegenüberliegende Quadranten des Ober- bzw. Untergebisses eingesetzt werden können. Aufgrund der für die Zähne in Okklusionsstellung vorbereiteten Aufstellung ist somit auch nach Einsetzen und Einpassen in den entsprechenden Kieferbereich eine Nachbearbeitung der Zähne praktisch nicht notwendig.

Eine besonders vorteilhafte Herstellungstechnik für das Fräsblocksystem ist die Möglichkeit, die ausgewählten Zähne in der Okklusionsstellung in dem ersten Bereich der Prothesenbasis vorpolymerisiert einzusetzen und dann erst bei dem Zusammenfügen mit dem zweiten Bereich der Prothesenbasis die Endpolymerisation vorzunehmen. Auf diese Weise wird erreicht, dass die jeweilige Ober- und Unterkieferprothese einstückig und form- und kraftschlüssig präpariert und hergestellt wird und somit ein geringeres Allergiepotential sowie eine höhere Verbundfestigkeit aufweist.

Weitergehende Ausbildungen der vorliegenden Erfindung sind Gegenstand der weiteren Unteransprüche.

Mit dem anmeldungsgemäßen Fräsblocksystem wird ermöglicht, dass eine Fixierung bzw. Kennzeichnung jeglicher Art für eine Nulllageübergabe bzw. Nullpunktsübergabe zur maschinellen CAD/CAM Bearbeitung vorgenommen werden kann. Dies ermöglicht eine enorme Zeitersparnis, insbesondere durch Wegfall des Abtastens, zur Erreichung der Lagepositionierung und verhindert Fehlbearbeitungen in einem CAD/CAM System.

Das Fräsblocksystem und das Verfahren zur Herstellung von Teil- oder Totalprothesen werden anhand der Zeichnungen nachstehend beispielhaft erläutert.

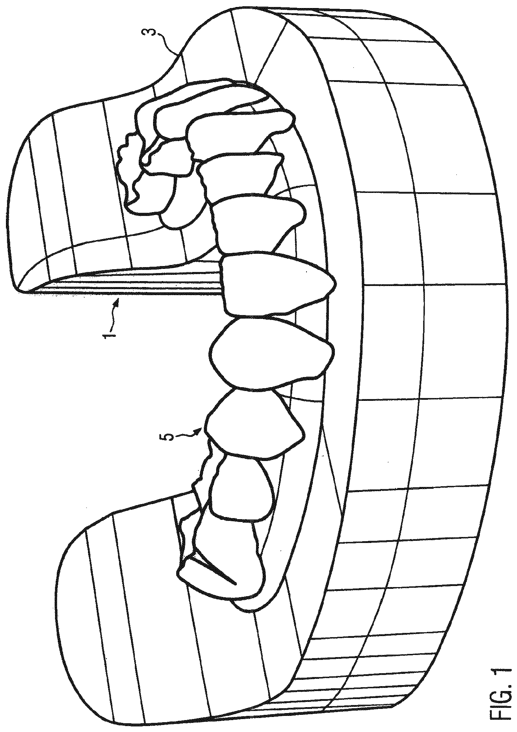

In Figur 1 ist ein Fräsblock 1 dargestellt, der eine Prothesenbasis 3 aufweist, die entsprechend der Kieferform bearbeitet wird, auf der bereits künstlich geformte Zähne 5 eines vollständigen Gebisses vorgesehen sind. Die künstlichen Zähne sind bereits so gearbeitet, dass die Zähne entsprechend einer Okklusionsstellung ausgerichtet sind und somit optimal mit dem nicht dargestellten Oberkiefer zusammenwirken können. In dieser Darstellung sind beispielsweise die Prothesenbasis und die Zähne einstückig ausgebildet, sodass der Herstellungsprozess vereinfacht wurde.

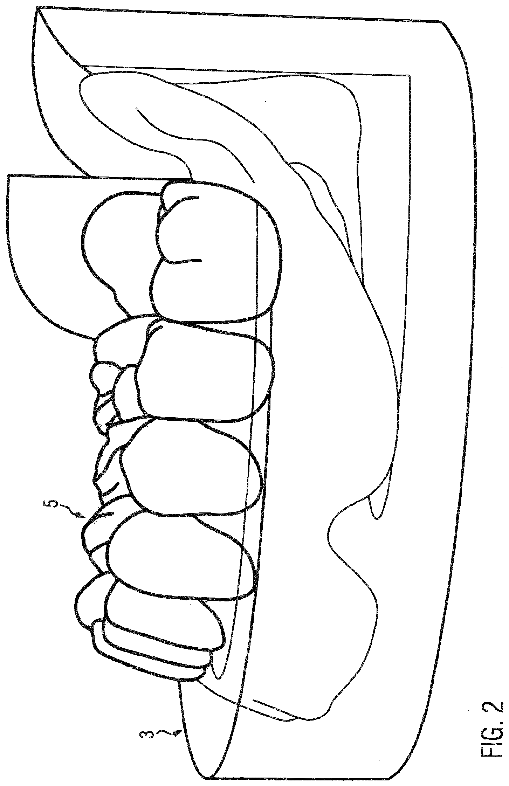

In Figur 2 ist die fertiggestellte Totalprothese des Unterkiefers dargestellt, wobei die Prothesenbasis 3 bereits an die Mundhöhle angepasst ist. Die Figur 2 zeigt deutlich, wie aus der Prothesenbasis die Form der Mundhöhle ausgefräst wird, wobei die Zähne 5, welche in Okklusionsstellung ausgerichtet sind, unbehandelt bleiben. Mit dem anmeldungsgemäßen Verfahren wird somit erreicht, dass lediglich durch Anpassung der Prothesenbasis eine vollständige Totalprothese vorliegt, deren Okklusion stimmig ist und kein weiterer Verfahrensschritt, wie beispielsweise Einpassen in einen Artikulator, erforderlich macht. Auch ein weiterer Verfahrensschritt, wie beispielsweise im Stand der Technik genannt, nämlich die nachträgliche Ausrichtung durch Aufbau der Zähne in Wachs, um die Okklusionsstellung zu erreichen, ist mit dem anmeldungsgemäßen Verfahren nicht erforderlich.

## Patentansprüche

1. Fräsblocksystem (1) zur Herstellung von Teil- oder Totalprothesen, umfassend eine ersten Fräsblock für das Obergebiss und einen zweiten Fräsblock für das Untergebiss, wobei jeder Fräsblock eine entsprechend der Kieferform zu bearbeitenden Prothesenbasis (3) aufweist, auf der künstlich geformte Zähne (5), vorzugsweise eines vollständigen Gebisses, vorgesehen sind, und die Zähne entsprechend gegebener Grunddaten nach vorbestimmten bzw. standardisierten gängigen Okklusionsprinzipien für die Okklusionsstellung aufgestellt sind.

2. Fräsblocksystem nach Anspruch 1, **dadurch gekennzeichnet, dass** die Prothesenbasis und die Zähne einstückig ausgebildet sind.

3. Fräsblocksystem nach einem der Ansprüche 1 oder 2, **dadurch gekennzeichnet, dass** die Prothesenbasis zweiteilig ausgestaltet ist, bestehend aus einem ersten, dem zu bearbeitenden Bereich und aus einem zweiten, dem die künstlich geformten Zähne aufweisenden Bereich, wobei vorzugsweise durch Polymerisation der erste und zweite Bereich zur Prothesenbasis verbunden sind.

4. Fräsblocksystem nach einem der Ansprüche 1 bis 3, **dadurch gekennzeichnet, dass** dieses als Materialien Keramiken wie Glaskeramik, leuzitverstärkte Keramik, aluminiumverstärkte Keramik, Oxidkeramik, Zirkonoxidkeramik, Infiltrationskeramik, Feldspatkeramiken oder Lithium-Disilikat-Keramik aufweist oder vorzugsweise als Materialien PMMA-Kunststoffe, Thermoplasten, Peek Nylon Composite, organisch sowie anorganisch gefüllte Kunststoffe, allergiearme Kunststoffe, ausbrennbare Materialien und/oder gesinterte Materialen aufweist.

5. Fräsblocksystem nach einem der Ansprüche 1 bis 4, **dadurch gekennzeichnet, dass** dieses für kombinierten Zahnersatz, insbesondere auf Metallbasis bzw. Metallkunststoffbasis bzw. Keramikbasis mit Kunststoffzähnen verwendbar ist.

6. Fräsblocksystem nach einem der Ansprüche 1 bis 5, wobei die Prothesenbasis jedes Fräsblocks entsprechend der Kontur einer Mundhöhle eines Patienten bearbeitbar ist.

7. Fräsblocksystem nach Anspruch 6, wobei die Zähne des Obergebisses und des Untergebisses entsprechend zur Bissregistrierung ausgerichtet sind.

8. Verfahren zur Herstellung von Teil- oder Totalprothesen, welches aus folgenden Schritten besteht:
a. Herstellen bzw. Bereitstellen eines ersten Fräsblocks für das Obergebiss und eines zweiten Fräsblocks für das Untergebiss, wobei der erste und der zweite Fräsblock jeweils eine Prothesenbasis aufweisen, auf der künstlich geformte Zähne, vorzugsweise eines vollständigen Gebisses, vorgesehen sind, wobei die Zähne entsprechend gegebener Grunddaten nach vorbestimmten bzw. standardisierten gängigen Okklusionsprinzipien für die Okklusionsstellung aufgestellt sind.
b. Bestimmen der Konturdaten der Mundhöhle eines Patienten;
c. Präparieren und/oder Bearbeiten des ersten und zweiten Fräsblocks, und zwar der Prothesenbasis, entsprechend der Konturdaten der Mundhöhle.

9. Verfahren nach Anspruch 8, wobei das Präparieren und/oder Bearbeiten des ersten und zweiten Fräsblocks mittels CAD/CAM Systemen erfolgt, und/oder vorzugsweise die Zähne nach den Aufstellverfahren Gysi, Gerber, APF, APF NT, TiF, Schreinemaker, Staub Cranial aufgestellt sind.

10. Verfahren nach einem der Ansprüche 8 oder 9, wobei die ausgewählten künstlich geformten Zähne auf einen zweiten Bereich der Prothesenbasis aufgestellt sind und dieser zweite Bereich mit einem ersten, dem zu bearbeitenden Bereich der Prothesenbasis zusammengefügt ist und vorzugsweise der erste und der zweite Bereich durch Polymerisation, durch Kleben, durch Pressen und/oder Verschmelzen zusammengefügt sind.

11. Verfahren nach einem der Ansprüche 8 bis 10, wobei die ausgewählten Zähne in der Okklusionsstellung im zweiten Bereich vorpolymerisiert und/oder anschließend mit dem ersten Bereich endpolymerisiert sind.

12. Verfahren nach einem der Ansprüche 8 bis 11, wobei die Prothesenbasis zur vereinfachten Einpassung in die Mundhöhle bzw. Anpassung an die Kieferform des Patienten flexibel ausgestaltet ist und vorzugsweise die flexible Prothesenbasis durch Erwärmen, Bestrahlung mit Licht oder durch chemische Reaktion mindestens zweier in der Prothesenbasis vorhandener Komponenten versteift bzw. verfestigt wird.

13. Verfahren nach dem Anspruch 12, **dadurch gekennzeichnet, dass** die palatinalen und vestibulären Bereiche der Prothesenbasis nicht bearbeitet werden.

## Claims

1. A milling block system (1) for the production of partial or complete dentures, comprising a first milling block for the upper dentition and a second milling block for the lower dentition, wherein each milling block has a denture base (3) to be machined according to the shape of the jaw, on which artificially formed teeth (5), preferably of a complete dentition, are provided, and the teeth are set up according to predetermined or standardized common occlusion principles for the occlusal position according to given basic data.

2. The milling block system according to claim 1, **characterized in that** the denture base and the teeth are formed in one piece.

3. The milling block system according to any one of claims 1 or 2, **characterized in that** the denture base is designed in two parts, consisting of a first area to be machined and a second area comprising the artificially formed teeth, the first and second areas preferably being connected by polymerization to form the denture base.

4. The milling block system according to any one of claims 1 to 3, **characterized in that** it has ceramics such as glass ceramics, leucite-reinforced ceramics, aluminum-reinforced ceramics, oxide ceramics, zirconium oxide ceramics, infiltration ceramics, feldspar ceramics or lithium disilicate ceramics as materials or preferably has PMMA plastics, thermoplastics, peek nylon composites, organically and inorganically filled plastics, low-allergen plastics, burn-out materials and/or sintered materials as materials.

5. The milling block system according to any one of claims 1 to 4, **characterized in that** it can be used for combined dentures, in particular metal-based or metal-resin-based or ceramic-based with resin teeth.

6. The milling block system according to any one of claims 1 to 5, wherein the denture base of each milling block can be machined according to the contour of an oral cavity of a patient.

7. The milling block system according to claim 6, wherein the teeth of the upper dentition and the lower dentition are correspondingly aligned for bite registration.

8. A method for the manufacture of partial or total prostheses, which consists of the following steps:
a. manufacturing or providing a first milling block for the upper dentition and a second milling block for the lower dentition, wherein the first and the second milling block each have a denture base on which artificially formed teeth, preferably of a complete dentition, are provided, wherein the teeth are set up according to predetermined or standardized common occlusion principles for the occlusal position in accordance with given basic data.
b. determining the contour data of a patient's oral cavity;
c. preparing and/or machining the first and second milling blocks, namely the denture base, according to the contour data of the oral cavity.

9. The method according to claim 8, wherein the preparation and/or machining of the first and second milling blocks is carried out by means of CAD/CAM systems, and/or preferably the teeth are set up according to the Gysi, Gerber, APF, APF NT, TiF, Schreinemaker, Staub Cranial set-up methods.

10. The method according to any one of claims 8 or 9, wherein the selected artificially formed teeth are set up on a second area of the denture base and this second area is joined to a first area of the denture base to be machined and preferably the first and the second area are joined by polymerization, by gluing, by pressing and/or by fusing.

11. The method according to any one of claims 8 to 10, wherein the selected teeth in the occlusal position are pre-polymerized in the second area and/or subsequently end-polymerized with the first area.

12. The method according to any one of claims 8 to 11, wherein the denture base is designed to be flexible for simplified fitting into the oral cavity or adaptation to the shape of the patient's jaw and preferably the flexible denture base is stiffened or solidified by heating, irradiation with light or by chemical reaction of at least two components present in the denture base.

13. The method according to claim 12, **characterized in that** the palatal and vestibular areas of the denture base are not machined.

## Revendications

1. Système de blocs de fraisage (1) pour la production de prothèses partielles ou complètes, comprenant un premier bloc de fraisage pour la denture supérieure et un deuxième bloc de fraisage pour la denture inférieure, dans lequel chaque bloc de fraisage comporte une base de prothèse (3) à usiner selon la forme de la mâchoire, sur laquelle sont pourvues des dents formées artificiellement (5), de préférence d'une denture complète, et les dents sont agencées selon des données de base fournies conformément à des principes d'occlusion communs prédéterminés ou standardisés pour la position occlusale.

2. Système de blocs de fraisage selon la revendication 1, **caractérisé en ce que** la base de prothèse et les dents sont formées en une seule pièce.

3. Système de blocs de fraisage selon l'une des revendications 1 et 2, **caractérisé en ce que** la base de prothèse est conçue en deux parties, constituées d'une première zone à usiner et d'une deuxième zone comportant les dents formées artificiellement, dans lequel les première et deuxième zones sont de préférence assemblées par polymérisation pour former la base de prothèse.

4. Système de blocs de fraisage selon l'une des revendications 1 à 3, **caractérisé en ce qu'**il comprend comme matériaux des céramiques telles que des vitrocéramiques, des céramiques renforcées à la leucite, des céramiques renforcées à l'aluminium, des céramiques d'oxyde, des céramiques d'oxyde de zirconium, des céramiques d'infiltration, des céramiques de feldspath ou des céramiques de disilicate de lithium, ou comprend de préférence comme matériaux des plastiques PMMA, des thermoplastiques, des composites de nylon PEEK, des plastiques à charge organique ou inorganique, des plastiques hypoallergéniques, des matériaux combustibles et/ou des matériaux frittés.

5. Système de blocs de fraisage selon l'une des revendications 1 à 4, **caractérisé en ce qu'**il est utilisable pour les prothèses dentaires combinées, en particulier à base métallique, à base métal-plastique ou à base céramique avec des dents en matière plastique.

6. Système de blocs de fraisage selon l'une des revendications 1 à 5, dans lequel la base de prothèse de chaque bloc de fraisage peut être usinée selon le contour de la cavité buccale d'un patient.

7. Système de blocs de fraisage selon la revendication 6, dans lequel les dents de la denture supérieure et de la denture inférieure sont alignées de manière correspondante à l'enregistrement de l'occlusion.

8. Procédé de fabrication de prothèses partielles ou complètes, comprenant les étapes suivantes :
a. fabrication ou fourniture d'un premier bloc de fraisage pour la denture supérieure et d'un deuxième bloc de fraisage pour la denture inférieure, dans lequel chacun des premier et deuxième blocs de fraisage comporte une base de prothèse sur laquelle sont pourvues des dents formées artificiellement, de préférence d'une denture complète, dans lequel les dents sont agencées selon des données de base fournies conformément à des principes d'occlusion communs prédéterminés ou standardisés pour la position occlusale ;
b. détermination des données de contour de la cavité buccale d'un patient ;
c. préparation et/ou usinage des premier et deuxième blocs de fraisage, à savoir la base de prothèse, conformément aux données de contour de la cavité buccale.

9. Procédé selon la revendication 8, dans lequel la préparation et/ou l'usinage du premier bloc de fraisage et du deuxième bloc de fraisage sont effectués à l'aide de systèmes CAD/CAM, et/ou de préférence les dents sont agencées selon les procédés d'agencement Gysi, Gerber, APF, APF NT, TiF, Schreinemaker ou Staub Cranial.

10. Procédé selon l'une des revendications 8 et 9, dans lequel les dents formées artificiellement sélectionnées sont agencées sur une deuxième zone de la base de prothèse, et cette deuxième zone est assemblée avec une première zone de la base de prothèse à usiner, et de préférence la première zone et la deuxième zone sont assemblées par polymérisation, par collage, par pressage et/ou par fusion.

11. Procédé selon l'une des revendications 8 à 10, dans lequel les dents sélectionnées sont pré-polymérisées dans la deuxième zone en position occlusale et/ou sont ensuite post-polymérisées avec la première zone.

12. Procédé selon l'une des revendications 8 à 11, dans lequel la base de prothèse est conçue pour être flexible afin de faciliter son insertion dans la cavité buccale ou son adaptation à la forme de la mâchoire du patient, et de préférence la base de prothèse flexible est rigidifiée ou durcie par chauffage, par irradiation lumineuse ou par réaction chimique d'au moins deux composants présents dans la base de prothèse.

13. Procédé selon la revendication 12, **caractérisé en ce que** les zones palatines et vestibulaires de la base de prothèse ne sont pas usinées.
